Europäisches Patentamt

European Patent Office

Office européen des brevets

(11) Publication number:

0 326 691
A1

## (12) EUROPEAN PATENT APPLICATION

(21) Application number: 88120914.2

(22) Date of filing: 14.12.88

(51) Int. Cl.4: C12N 15/00

(30) Priority: 19.12.87 JP 320113/87

(43) Date of publication of application:
09.08.89 Bulletin 89/32

(84) Designated Contracting States:
AT BE DE FR GB IT

(71) Applicant: Tosoh Corporation
4560, Oaza Tonda
Shinnanyo-shi Yamaguchi-ken(JP)

(72) Inventor: Oda, Akihiko
3-18-6, Naka-machi
Machida-shi Tokyo(JP)
Inventor: Tazaki, Seiichi
5780, Seya-machi Seya-ku
Yokohama-shi Kanagawa-ken(JP)
Inventor: Fujii, Tatsushi
2-7-3, Tachibanadai Midori-ku
Yokohama-shi Kanagawa-ken(JP)

(74) Representative: Patentanwälte Grünecker,
Kinkeldey, Stockmair & Partner
Maximilianstrasse 58
D-8000 München 22(DE)

(54) Method of controlling genetic expression.

(57) Method for controlling genetic expression comprising cultivation Entrobacteria transformed by a vector carrying regulatory genes of a least tryptophan promoter, lactose operator and a gene which is coded for herterogenic protein located in the downstream of the regulatory genes, in a culture medium containing glucose and/or fructose.

## METHOD OF CONTROLLING GENETIC EXPRESSION

Background of the Invention:

Field of the Invention:

The present invention relates to a method for producing a required protein by Entrobacteria (especially E. Coli) using the genetic recombination technique. More particularly, this invention relates to a method for controlling expressions of genes which contain tryptophan promoter and lactose operator as regulatory genes (hereinafter the regulatory genes are designated as tac).

Description of the Related Art:

The tac is a hybrid regulatory gene system comprising tryptophan promoter and lactose operator, and is known as being capable of strongly expressing the genes located downstream.

Due to the strong expressing ability of the tac, when E. Coli transformed by a vector in which a sequence of genes coding for the object protein is combined with the tac is used, a heterogenic protein which does not occur under the natural condition may be produced in a larger amount than otherwise.

If the object protein is secreted in the culture medium immediately after production, there will be no problem. However, in case the protein is a heterogenic protein which is not secreted in the culture medium but is accumulated in E. Coli and, for example, forms granules of precipitate, growth of E. Coli will be markedly impeded as the amount of production of the heterogenic protein increases.

Solution of this problem requires to control production of the object protein. Thus, it is required to cultivate the recombined E. Coli while the production of the object protein is inhibited, or in other words, expression of the gene which is coded for the object protein is inhibited, and then to release the inhibition when E.Coli has grown up to a sufficient amount.

The tac is inhibited by a repressor protein of the lactose regulatory genes produced by E. Coli under the natural condition. However, the repressor protein of the lactose regulatory genes which ordinary E.Coli produce is primarily produced only in a minute amount for the purpose to inhibit the lactose regulatory genes which E.Coli holds in nature. Therefore the amount is too small to inhibit the expression of the tac which is introduced in the recombined E.Coli.

In ordinary E.Coli, the repressor protein which inhibits the expression of lactose regulatory gene is coded by the gene called lacl. However, if the tac is used for the expression of heterogenic object protein, the gene called $lacl^q$ should be called for which can produce more than ten times as much repressor protein than that lacl does, for the reason mentioned above.

The tac can be released from the condition of inhibition by interfering with the repressor protein produced by the $lacl^q$ to combine with the lactose operator. In case of a variant which produces more than ten times as much repressor protein as ordinary E. Coli does, a chemical substance such as isopropyl-$\beta$-D-thiogalactopyranoside (hereinafter designated as IPTG) should be added.

As described above, use of the tac as regulatory gene is desirable with respect to the productivity of the object protein, but on the other hand it is necessary to use a E. Coli variant $lacl^q$ and also to add IPTG, when the object protein is accumulated in E. Coli. The present inventors continued their investigations on the background mentioned above, and found that the presence of glucose and/or fructose inhibits the expression of tac (the expression of tac means the expression of the gene that is coded for the object protein, or expression of object protein itself), to complete the present invention.

Summary of the Invention:

The present invention provides a method for controlling genetic expression comprising cultivation of E.Coli transformed by a vector carring at least tryptophan promoter, lactose operator and a gene which codes for heterogenic protein located in the downstream of said regulatory genes, in a culture medium containing glucose and/or fructose.

Detailed Description of the Invention:

The present invention can be applied to the cultivation of E.Coli transformed by a vector where the genes that code for such soluble and insoluble heterogenic proteins, which after expressed in E.Coli is not secreted in the culture medium but accumulated, as tissue plasminogen activator, human growth hormone, insulin, prourokinase, and bovine serum albumin are combined with the tac.

The vector is not particularly restricted so long as it is capable of transforming E.Coli and does not contain $lacl^q$ introduced such as pBR322 or pMB9. For the vectors, reference may be made to literatures such as, for example, "Molecular Cloning", Cold Spring Harbor Laboratory, 1982 and "Vector DNA", Kodansha, 1986. Construction of the genetic sequence and the transduction in a vector may be made by known arts. Such strain as HB101 or C600 which does not contain lacl$^q$ but contains lacl may be used, for which reference may be made to "Molecular Cloning", Cold Spring Harbor Laboratory, 1982.

The amount of glucose and fructose to be added may usually be that which is consumed out by E.Coli up to the end of the exponential growth phase in the culture medium employed. However, according to the operator s convenience, the amount may be that presumably consumed when E.Coli has grown up to the desired amount. Glucose and fructose may be used either alone or in combination. In determining the amount of flucose and/or fructose to be added considered is growth yield of E.Coli for the carbon source such as glucose and/or fructose which is observed when untransformed E.Coli is cultivated, because the expression of the gene for object heterogenic protein is inhibited in the recombined E.Coli as long as glucose and/or fructose are present.

Further, in case of present of other carbon sources in culture medium, relation between consumption rate for glucose and/or fructose and that for the other carbon sources, mentioned below, is considered

The culture medium may be the same one as that for usual cultivation of E.Coli as long as it contains carbon sources, nitrogen sources and metal ions in an amount sufficient for the growth of E.Coli. At the moment when glucose and fructose which inhibit the expression of tac are consumed and tac is expressed, a carbon source necessary for E.Coli to produce the object heterogenic protein must be present. This requirement can be met by adding intermediates in the glycolytic series such as glycerol, intermediates in the TCA cycle such as acetic and pyruvic acids. The combination of glucose and/or fructose with other carbon source which will be consumed in expression of tac may be determined in consideration of the difference of consumption rate of E.Coli. That is those carbon sources can be used in combination with glucose and/or fructose in this invention which are not consumed when glucose and/or fructose is present or consumed in an incomparably small speed than glucose or fructose are. A combination of glucose and glycerol is an example. In a culture medium containing this combination, glucose that inhibits tac is consumed first and then glycerol is consumed while E.Coli produces a heterogenic protein. This combination is a preferable one and the amount of glucose to be added can be determined from the growth yield of E.Coli for glucose which is culculated when E.Coli is caltivated only with glucose. The combination of glucose and glycerol is recommended in this invention. However, depending on circumstances where other carbon sources are used which ar comsumed by E.Coli incomparably faster than glucose and fructose, said other carbon sources may be added to the culture medium after glucose and/or fructose have been consumed.

The present inventors observed in the case when glucose and glycerol were used as carbon source that the difference of (A) the dry weight in grams of bacteria per liter of culture medium when the inhibition of tac is released and (B) the dry weight in grams of bacteria primarily applied per liter of culture medium, the difference being the increased amount of bacteria per liter of the culture medium , was related to (C) the glucose in grams added per liter of the culture medium as

$$C / (A - B) = 2 \sim 3.$$

Since the concentration of primarily applied bacteria is usually minute as compared with the concentration at the end of exponential growth phase of E.Coli or the desired concentration of bacteria, the preferable amount of glucose to be added, insofar as it is used as carbon source together with glycerol, is reasonably considered to be 2 to 3 times as much as the desired amount of E.Coli.

In the present invention, other conditions on the culture, that is for example temperature of culture and pH of the culture medium, may be as they are in known arts.

The present invention permits the tac to be used in combination with a strain other than the lacl$^q$ variant containing lacl$^q$ which was hitherto necessary for inhibiting the strong expressing ability of tac. Moreover, the process so far considered necessary in which an expression inducing substance such as IPTG is added after E.Coli has fully grown could be omitted by adding beforehand a predetermined amount of glucose and/or fructose which will be consumed up to the full growth of E.Coli.

Brief Explanation of Drawing:

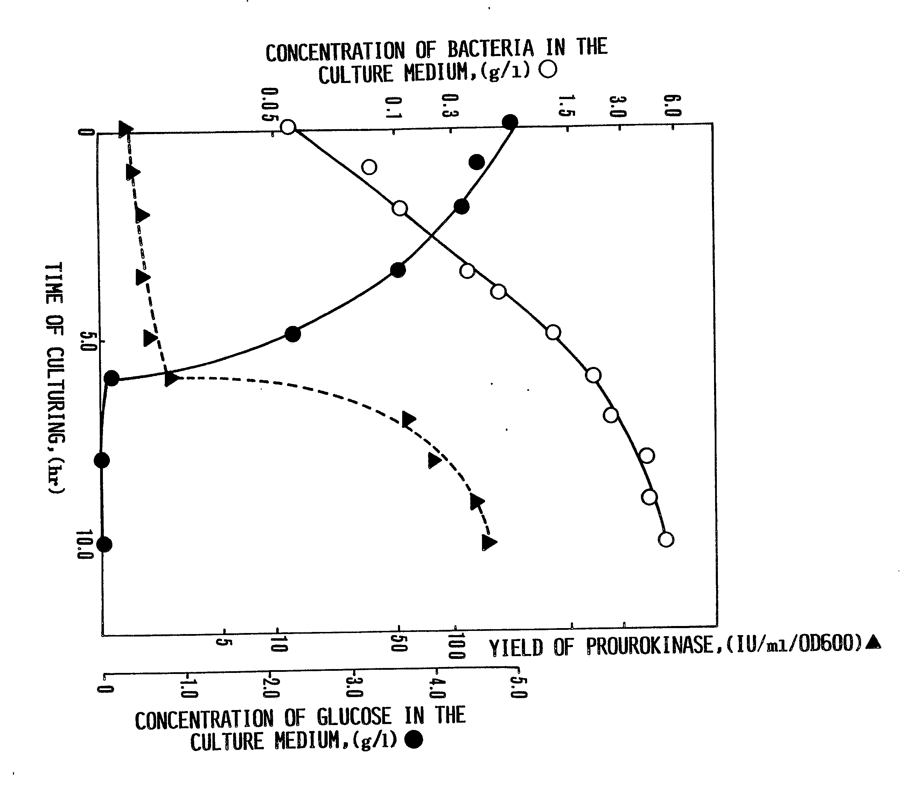

Fig. 1 shows results of Example 1. Expression of prourokinase is hardly observed while glucose exists in the culture medium.

Detailed Description of the Preferred Embodiments:

Example is shown below for detailed explanation of this invention, but this invnetion is not limited to the example.

Example 1

A plasmid comprising the genetic sequence in which a gene that codes for prourokinase is combined with tac (the plasmid was obtained from E.Coli deposited to Governmental Research Institute for Microbiological Industries in Japan as International deposited No. 970 and No. 971) was used to transform E.Coli not having lacl$^q$ (HB101). Seed culture containing 0.6 g - dry -E.Coli per 1 liter was transferred culture medium containing 1 % of glycerol, 2 % of yeast extract, 0.7 % of $NH_2HPO_4$, 0.3 % of $KH_2PO_4$, 0.1 % of $NH_4Cl$, 0.05 % of NaCl, 30 mM of $MgSO_4$, 30 mM of $CaCl_2$ and 0.05 % of ampicillin (where % means percentages by weight per liter of the culture medium). In addition to the mentioned components, glucose was further added to the culture medium in an amount of 5 g/l so that the glucose was consumed out when the dry weight of E.Coli grew up about 2 g/l. While E.Coli was cultivated at 30° C, estimations were made with amount of bacteria, remaining amount of glucose, and expressed amount of prourokinase. The remaining amount of glucose was estimated with a sugar analyzer YSI23A of Yellow Spring Instruments Co. In the estimation of prourokinase, E.Coli was sonicated to solubilize prourokinase, according to the process disclosed in Japanese Laid-Open Patent Application No. Sho- 59-161321. The reactivated prourokinase obtained was treated with plasmin to produce urokinase, the activity of which represented the estimation of original prourokinase. The activity measurement of the urokinase was made by the process of T. Kohno et al (Biotechnology, 628, 1984) using a synthesized substrate S-2444 (produced by Daiichi Chemicals Co.) and the result was expressed by the activity per turbidity at 600 nm.

**Claims**

1. Method for controlling genetic expression comprising cultivation Entrobacteria transformed by a vector carrying regulatory genes of at least tryptophan promoter, lactose operator and a gene which is coded for heterogenic protein located in the downstream of said regulatory genes, in a culture medium containing glucose and/or fructose.

EP 0 326 691 A1

European Patent Office

EUROPEAN SEARCH REPORT

Application Number

EP 88 12 0914

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PROC. NATL. ACAD. SCI. USA, vol. 80, January 1983, pages 21-25; H.A. DE BOER et al.: "The tac promoter: A functional hybrid derived from the trp and lac promoters" <br> * Whole document * <br> --- | 1 | C 12 N 15/00 |
| Y | CHEMICAL ABSTRACTS, vol. 78, no. 9, 5th March 1973, page 269, abstract no. 55246a, Columbus, Ohio, US; A.E. SILVERSTONE et al.: "Polycistronic effects of catabolite repression on the lac operon", & J. BACTERIOL. 1972, 112(3), 1184-92 <br> * Abstract * <br> --- | 1 | |
| Y | JOURNAL OF BACTERIOLOGY, vol. 159, no. 3, September 1984, pages 832-836, American Society for Microbiology; H. GOLDIE: "Regulation of transcription of the Escherichia coli phosphoenolpyruvate carboxykinase locus: studies with pck-lacZ operon fusions" <br> * Page 834, column 1, line 19 - page 835, column 2, line 3; figure 2 * <br> --- | 1 | TECHNICAL FIELDS SEARCHED (Int. Cl.4) <br><br> C 12 N |
| Y | CHEMICAL ABSTRACTS, vol. 70, 1969, page 104, abstract no. 75362y, Columbus, Ohio, US; I. PASTAN et al.: "Role of the lac promotor locus in the regulation of beta-galactosidase synthesis by cyclic 3',5'-adenosine monophosphate", & PROC. NAT. ACAD. SCI. U.S. 1968, 61(4), 1336-42 <br> * Abstract * <br> ---       -/- | 1 | |

The present search report has been drawn up for all claims

| Place of search | Date of completion of the search | Examiner |
|---|---|---|
| THE HAGUE | 21-04-1989 | PULAZZINI A.F.R. |

European Patent Office

Application Number

EP 88 12 0914

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | JOURNAL OF BACTERIOLOGY, vol. 146, no. 1, April 1981, pages 149-154; E. JOSEPH et al.: "Regulation of galactose operon expression: glucose effects and role of cyclic adenosine 3',5'-monophosphate" | | |
| A | CHEMICAL ABSTRACTS, vol. 107, no. 9, 31st August 1987, page 365, abstract no. 73964e, Columbus, Ohio, US; N.G. RAY et al.: "Regulation of lac operon expression in mixed sugar chemostat cultures", & BIOTECHNOL. BIOENG. 1987, 29(8), 1003-14 | | |

TECHNICAL FIELDS SEARCHED (Int. Cl.4)

The present search report has been drawn up for all claims

| Place of search | Date of completion of the search | Examiner |
|---|---|---|
| THE HAGUE | 21-04-1989 | PULAZZINI A.F.R. |

EPO FORM 1503 03.82 (P0401)